Europäisches Patentamt

European Patent Office

Office européen des brevets

(11) Publication number: **0 395 997**
**A1**

# (12) EUROPEAN PATENT APPLICATION

(21) Application number: **90107750.3**

(51) Int. Cl.5: **A61B 17/34, A61B 19/00**

(22) Date of filing: **24.04.90**

(30) Priority: **05.05.89 US 348613**

(43) Date of publication of application:
**07.11.90 Bulletin 90/45**

(84) Designated Contracting States:
**AT BE CH DE DK ES FR GB GR IT LI LU NL SE**

(71) Applicant: **Becton Dickinson and Company**
**One Becton Drive**
**Franklin Lakes New Jersey 07417-1880(US)**

(72) Inventor: **Houghton, Frederick C.**
**18 Maple Avenue**
**Sussex, New Jersey(US)**
Inventor: **Gregg, Joseph J.**
**65 Roosevelt Avenue**
**Hasbrouck Heights, New Jersey(US)**

(74) Representative: **Selting, Günther, Dipl.-Ing. et**
**al**
**Patentanwälte von Kreisler, Selting, Werner**
**Deichmannhaus am Hauptbahnhof**
**D-5000 Köln 1(DE)**

(54) **Electrically insulated breast lesion localization device.**

(57) An electrically insulated breast lesion localizing device (26) includes a cannula (21) having a proximal end (22), a distal end (23) and a lumen (25) therethrough. An elongate metallic localizer (26) having a proximal portion, a distal portion (29) and a resilient anchor portion (31) at the distal portion. The localizer is movably positioned within the lumen so that the proximal portion of the localizer extends outwardly from the proximal end of the cannula. The resilient anchor portion is deflected by contact with the lumen so that moving the cannula proximally with respect to he localizer causes the anchor portion to project outwardly from the distal end of the cannula and to spring to a size larger than the distance across the lumen. A coating is provided on the distal portion of the localizer for helping to insulate the localizer from the effects of electrical current from electrosurgical knives.

*FIG-4*

# ELECTRICALLY INSULATED BREAST LESION LOCALIZATION DEVICE

## BACKGROUND OF THE INVENTION

### 1. Field of the Invention.

The present invention relates to a breast lesion localization device and more particularly concerns a breast lesion localizing device with an electrically insulated localizer or marker.

### 2. Description of Related Information.

Widely used mammography techniques are capable of detecting breast lesions, many of which are nonpalpable. It is important to accurately mark these lesions for subsequent surgical procedures so that the lesion itself may be removed or operated upon without the removal of substantial amounts of non-effected tissue and to avoid the risk of not removing all of the lesion. The nature of breast tissue and the movement of the breasts relative to the orientation of the patient's body make it substantially impossible to accurately locate the lesion from marks made on the surface of the breast.

Needles, such as hypodermic needles have been used to mark the breast lesions. However, the smooth side wall of a hypodermic needle allows it to move from its initial position marking the lesion, and to possibly be inadvertently removed from the breast between the time of the mammography and the surgical procedure.

Kopans and DeLuca, in their March, 1980 article in Radiology teach a technique for localizing lesions using a hook wire having a 0.03cm diameter in conjunction with a 20 gauge spinal needle. The hook wire is bent over itself, at its distal end, to form a loop, and is then placed inside the needle cannula. The needle cannula is placed in the breast to mark the lesion. At this time the needle is withdrawn from the breast tissue leaving the hook wire, which has sprung open, to act as an anchor marking the lesion. The proximal end of the wire protrudes externally from the breast tissue.

More and more surgeons are using electrosurgical knives in surgical procedures. Such an electrosurgical knife is taught by Cartmell in U.S. Patent No. 4,800,878. Cartmell teaches an improved knife with an alarm system for alerting the physician of surges in the electrical system. Cartmell comments that the relatively small contact area between the tissue and the active electrode tip of the electrosurgical knife causes a concentration of current (high current density) that heats the tissue, thus allowing the electrosurgical cutting of the tissue.

Mammographic techniques have allowed the detection of nonpalpable breast lesions. Wire breast lesion markers have allowed the lesion to be marked during the mammographic technique for subsequent removal by the surgeon using a cutting instrument which may be an electrosurgical knife. It is believed that an electrosurgical knife used to remove a breast lesion marked with a wire marker may contact or otherwise induce a current in the marker causing the marker itself to be severed or to be heated by the current to possibly damage flesh in the patient. Although the prior art has allowed improved detection of breast lesions, improved marking of breast lesions, and improved removal using an electrosurgical device, there is still a need for a breast lesion localization device which will minimize any possible negative effects of using an electrosurgical device in the proximity of an electrically conducting wire localizer or marker.

## SUMMARY OF THE INVENTION

An electrically insulated breast lesion localizing device of the present invention includes a cannula having a proximal end, a distal end and a lumen therethrough. An elongate metallic localizer having a proximal portion, a distal portion and a resilient anchor portion at the distal portion is movably positioned within the lumen so that the proximal portion of the localizer extends outwardly from the proximal end of the cannula. The resilient anchor portion is deflected by contact with the lumen so that moving the cannula proximally with respect to the localizer causes the anchor portion to project outwardly from the distal end of the cannula and to spring open to a size larger than the distance across the lumen. A coating is provided on the distal portion of the localizer for helping to insulate the localizer from effects of electrical current from electrosurgical knives.

In accordance with the preferred embodiment of the present invention, an electrically insulated breast lesion localizing device includes an elongate cannula having a proximal end, a sharp distal end and a lumen therethrough. An elongate metal wire localizer having a proximal portion, a distal portion and a resilient anchor portion at the distal portion, formed by bending the distal portion in a proximal direction, is movably positioned within said lumen. The localizer is positioned so that the anchor portion is adjacent to the distal end of the cannula and the proximal portion of the localizer extends outwardly from the proximal end of the cannula. The resilient anchor portion of the localizer is deflected by contact with the lumen so that moving the cannula proximally with respect to the localizer causes the anchor portion to project outwardly from the distal end of the cannula and spring to a size larger than the distance across the lumen. An applied insulating coating is provided on the distal portion of the localizer for helping to insulate the localizer from the effects of electrical current from electrosurgical knives used for removing lesions. A hub is provided at the proximal end of the cannula to facilitate handling and positioning the cannula. The hub includes a passageway therethrough wherein the passageway is in communication with the lumen so that the localizer can pass through the passageway and the lumen.

## BRIEF DESCRIPTION OF THE DRAWINGS

Fig. 1 is a side elevation view of the breast lesion localizing device of the present invention;

Fig. 2 is a side elevation view of the distal end of the localizing device of Fig. 1,

Fig. 3 is a side elevation view of the proximal end of the localizing device of Fig. 1;

Fig. 4 is an enlarged partial cross-sectional view of the localization device of Fig. 2 taken along lines 4-4;

Fig. 5 is a side elevation view of the localizer outside of the cannula;

Fig. 6 is an enlarged cross sectional view of the localizer of Fig. 5 taken along lines 6-6;

Fig. 7 is a side elevational view of an alternative localizer;

Fig. 8 is a side elevational view of another alternative localizer having an elongate rigidifying member attached to its distal end;

Figs. 9-11 illustrate the use of the electrically insulated breast lesion localizing device of the present invention from the positioning of the distal end of the cannula in the lesion, to the removal of the cannula leaving the resilient anchor portion of the localizer in the lesion; and

Fig. 12 illustrates an alternative embodiment of the localizer positioned in a breast lesion.

## DETAILED DESCRIPTION

While this invention is satisfied by embodiments in many different forms, there are shown in the drawings and will herein be described in detail embodiments of the invention with the understanding that the present disclosure is to be considered exemplary of the principles of the invention and is not intended to limit the invention to the embodiments illustrated. The scope of the invention will be measured by the appended claims and their equivalents.

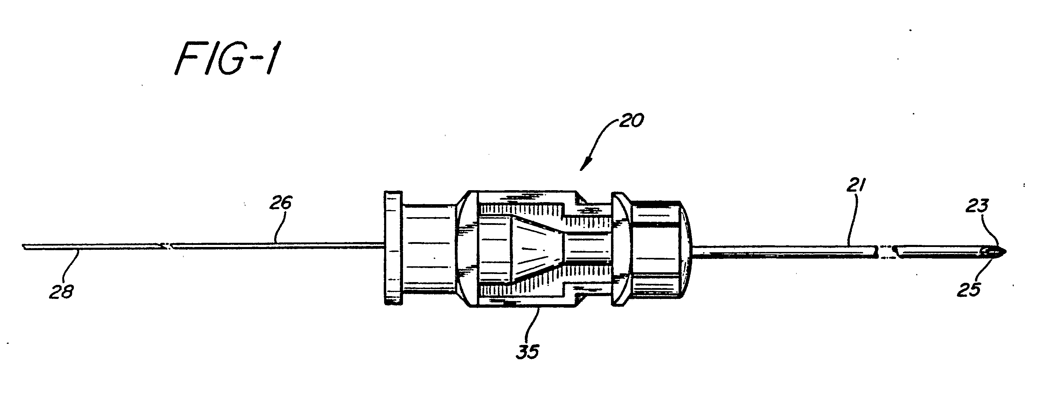

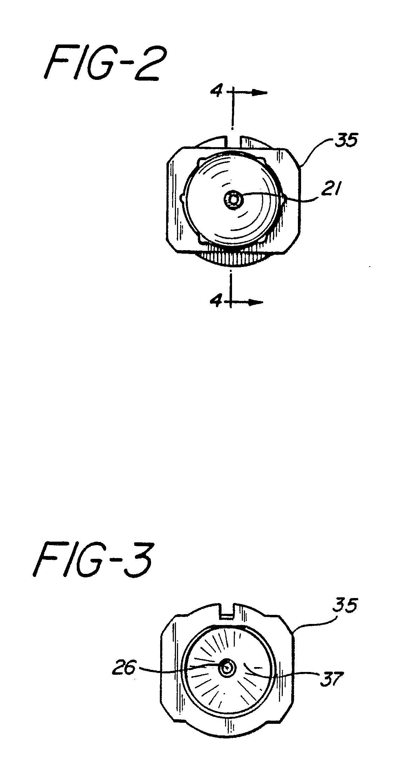

Adverting to Figs. 1-6 an electrically insulated breast lesion localization device 20 includes an elongate cannula 21 having a proximal end 22, a sharp distal end 23 and a lumen 25 therethrough.

For the purposes of the description of the present invention, the term "distal end" is meant to refer to the end furthest from the person holding the breast lesion localizing device, whereas the term "proximal end" is meant to refer to the end closest to the holder of the device.

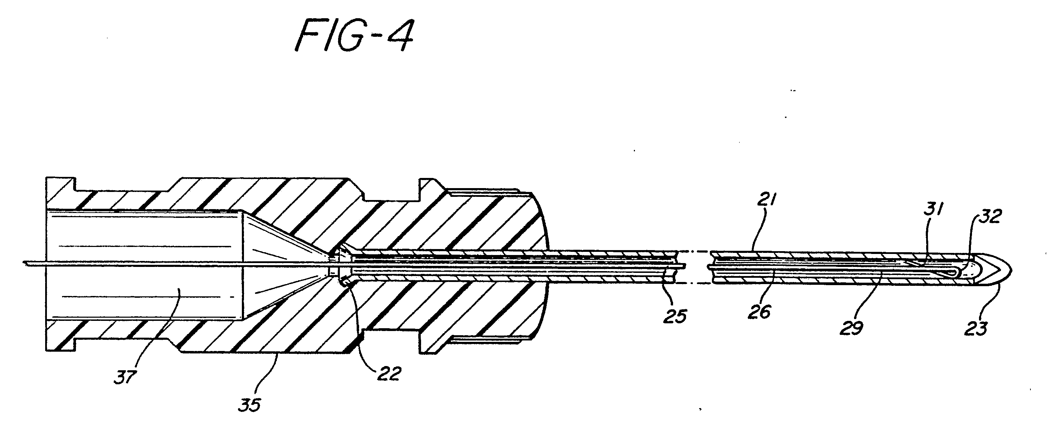

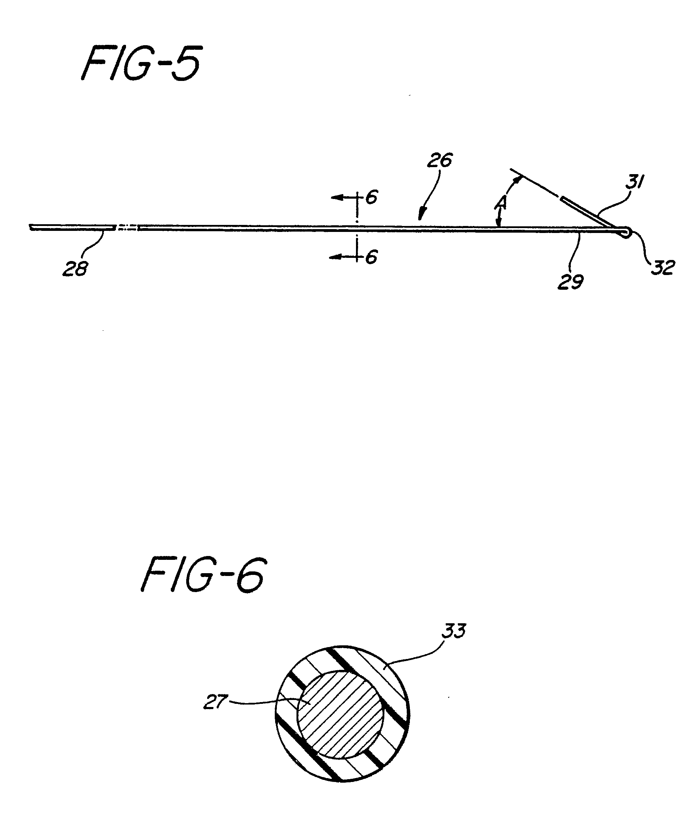

An elongate localizer or marker 26, formed of metal wire 27, includes a proximal portion 28, a distal portion 29 and a resilient anchor portion 31 at distal portion 29. The resilient anchor portion is formed by bending the distal portion of the localizer in a proximal direction. In this preferred embodiment the anchor portion is formed by bending the distal portion of the localizer more than 180° to form a bent loop portion 32 wherein the longitudinal axes of the portions of the distal portion adjacent to bent loop portion 32 cross at angle A which is desirably between 5° and 85° and preferably between 10° and 50°. Localizer 26 is movably positioned within the lumen so that anchor portion 31 of the localizer or marker is adjacent to distal end 23 of the cannula and the proximal portion of the localizer extends outwardly from the proximal end of the cannula. The resilient anchor portion of the localizer as illustrated in Fig. 5 is deflected by contact with the lumen, when it is placed within the lumen, as illustrated in Fig. 4. Moving the cannula proximally with respect to the localizer causes the anchor portion to project outwardly from the distal end of the cannula and to spring to a size larger than the distance across the lumen.

An applied coating of insulating material 33 is applied at least to distal portion 29 of the localizer and in this preferred embodiment is applied to the entire surface of metal wire 27 of the localizer.

A hub 35 at the proximal end of the cannula is provided to facilitate handling and positioning of the cannula. Hub 35 includes a passageway 37 therethrough. The passageway is in communication with lumen 25 so that the localizer can pass through the passageway and the lumen. It is preferred that the lumen have a diameter within the range of 0.7mm to 1.0mm In this embodiment the cannula is a 19 gauge cannula having an inside diameter of approximately 0.8mm. The localizer or marker, including applied coating 33, has a total overall diameter of between about 0.25mm and 0.35mn. In this embodiment the underlying wire is preferably made of stainless steel and has a diameter within the range of about 0.2mm to 0.3mn.

An important feature of the instant invention is applied coating 33 which is intended to help insulate the localizer from the effects of electrical current from electrosurgical knives so that the electrical energy of the electrosurgical knife will be directed primarily at tissue cutting and/or cauterizing rather than to excite or heat the localizing wire which could damage the wire or injure viable breast tissue not intended to be removed.

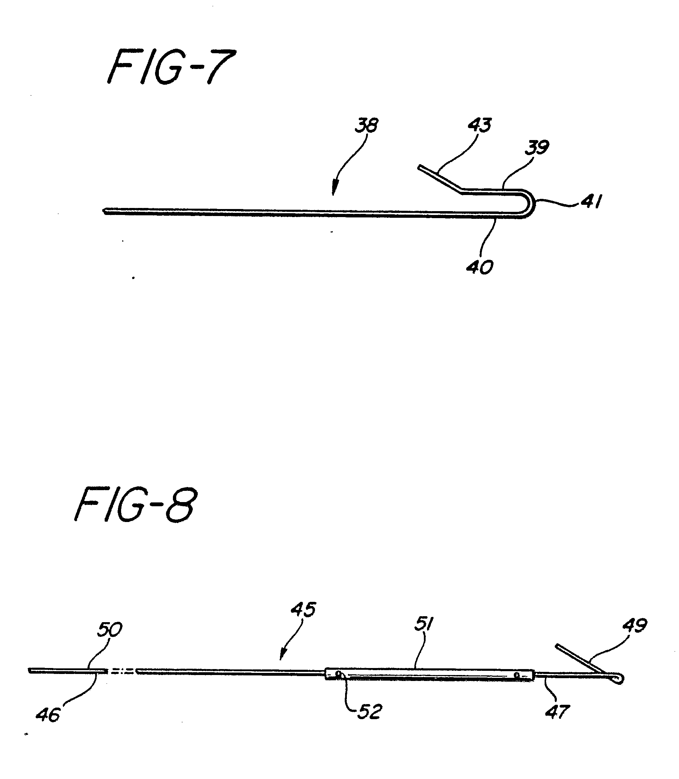

Referring now to Fig. 7, an alternative locali zer or marker 38 is illustrated. Localizer 38 is capable of functioning in conjunction with the cannula and hub assembly of the embodiment of Figs. 1-6. Localizer 38 includes an alternative resilient anchor portion 39 which is formed by bending distal portion 40 of the localizer over itself to form u-shaped structure 41 and then forming outwardly projecting dog leg 43. The anchor portion of this alternative localizer will compress when placed in the right cannula such as cannula 21 and will spring back to approximately its original shape, as illustrated in Fig. 7, upon removal of the cannula to help anchor the localizer in the breast lesion. This alternative anchor portion differs from the preferred anchor portion of Fig. 5 in that the wire is not bent over itself. It will be apparent to one skilled in the art that there are numerous forms the distal end of the localizer can take which will provide for an anchor portion which fits within a needle cannula and is capable of springing to a larger size upon release upon the cannula. All of these various forms for the distal end of the localizer are within the purview of the instant invention with the embodiments described herein be representative of these many possibilities.

Referring now to Fig. 8, another alternative embodiment of a localizer or marker 45 is illustrated. Localizer 45 is substantially identical to localizer 26 of the embodiments of Figs. 1-6 having a proximal portion 46, a distal portion 47 and a resilient anchor portion 49. The localizer includes a metal wire core having an applied insulating coating 50 on its outside diameter. Localizer 45 also includes an elongate rigidifying member 51 at distal portion 47 of the localizer for stiffening the localizer and improving the palpability of the localizer. In this embodiment, the elongate rigidifying member comprises an elongate stainless steel tube having an inside diameter (not shown) slightly larger than the outside diameter of the coated localizer so that the rigidifying member may be slid to the proper position on the coated wire and crimped at points of compression 52 to attach it to the localizer. It is within the purview of this embodiment to include an elongate rigidifying member made of insulating material such as plastic so that said coating comprises said rigidifying member. It is also within the purview of the present invention to attach the rigidifying member to the wire and to coat this assembly entirely or partially with an insulating coating. The embodiments of the electrically insulated localizer utilizing a rigidifying member include but are not limited to a rigidifying member made of conductive material such as metal, made of insulating material or coated with insulating material.

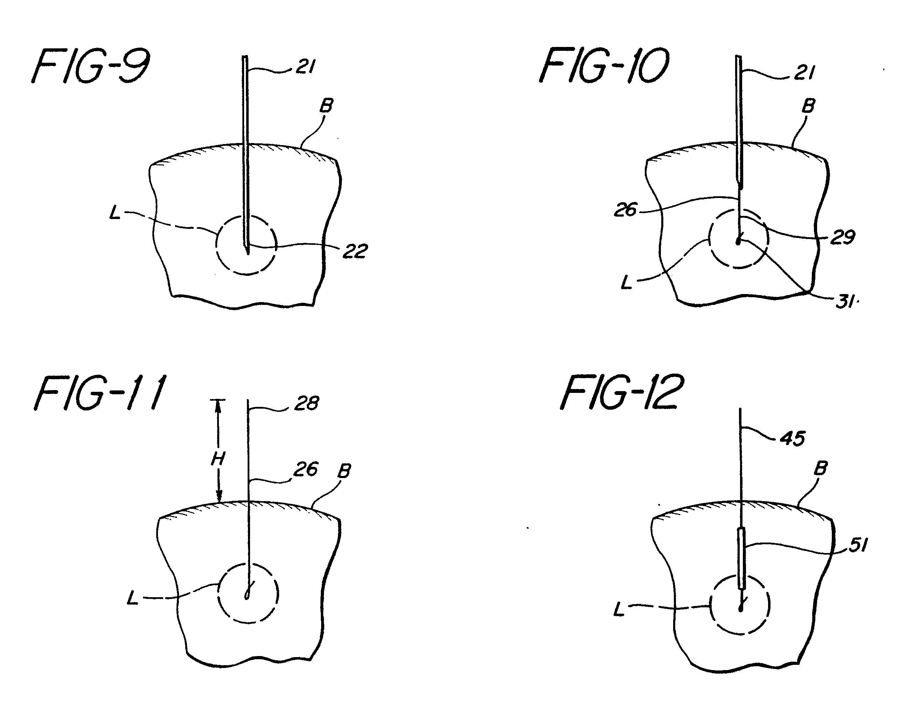

In use, as best illustrated in Figs. 9-11, the electrically insulated breast lesion localizing device of the instant invention is used in conjunction with a mammography apparatus which is capable of detecting lesion L. The instant invention is especially useful for marking a lesion which is nonpalpable to help guide the surgeon in removing the suspected lesion portion of the breast and not viable tissue outside of the lesion. In use, the mammography device locates lesion L. Then the breast lesion localizing device of the instant invention is caused to pierce the skin of breast B and penetrate to the lesion so that sharp distal end 22 of the cannula is in the lesion or at a predetermined position with respect to the lesion, as best illustrated in Fig. 9. When proper placement is verified, cannula 21 is withdrawn from the breast tissue leaving localizer 26 within the breast. Withdrawal of the cannula causes resilient anchor portion 31 of localizer 26 to project outwardly from the distal end of the cannula and to spring open to a larger size than the distance across the lumen or the inside diameter of the cannula if the cannula is circularly shaped. The springing open of anchor portion 31 causes it to act as an anchor and to resist movement from its placed position. After complete removal of a cannula, a length of localizer 26 projects outwardly from the breast. This portion may be cut to a predetermined length so that if the localizer shifts position or moves the surgeon will be able to determine this by measuring the free length H, at the time of surgery. Also, when using a very fine wire localizer, the free length H may be bent to be flush with the surface of the breast and taped thereon to help anchor the wire between the surface of the breast and the lesion during the time between placement and surgery.

Fig. 12 illustrates the alternative localizer 45 positioned in breast lesion L. The rigidifying member 51 is extremely helpful if a very fine wire is used in the localizer because it provides structural integrity and improved palpability.

The cannula of the present invention can be constructed of a wide variety of materials with metal such as stainless steel being preferred. The hub can be constructed of a wide variety of rigid materials with thermoplastic materials such as polypropylene, polyethylene and/or polystyrene being preferred. A wide variety of insulating materials are suitable for the applied coating of the localizer with plastic material such as polyvinyl chloride and polytetrafluoroethylene being preferred. Numerous methods of coating are known to those skilled in the art and include dipping in plastisol liquid and subsequent curing, and coating with ionized particles of the plastic material which are subsequently heated to fuse and form a coating on the underlying localizer structure. The preferred material for the localizer is metallic wire made of stainless steel. The cannula and the hub may be joined together using various methods known in the art such as medical grade adhesives including ultraviolet and heat curable adhesives and by mechanical interference fit including physically deforming or flaring the needle cannula after it is properly positioned within the hub. It is necessary that the breast lesion localizing device of the present invention be sterile when used. Accordingly, all components used in the device should be chosen to withstand the sterilization process being utilized.

Thus, it can be seen that the present invention provides a simple, straight-forward, reliable, easily fabricated, electrically insulated breast lesion localizing device which includes an insulating coating on the localizer which helps to in sulate the localizer from the effects of electrical current from electrosurgical knives used in the proximity of the localizer for removing lesions.

## Claims

1. An electrically insulated breast lesion localizing device comprising:
an elongate cannula having a proximal end, a distal end and a lumen therethrough;
an elongate metallic localizer having a proximal portion, a distal portion and a resilient anchor portion at said distal portion, said localizer being movably positioned within said lumen so that said anchor portion is adjacent to said distal end of said cannula and said proximal portion extends outwardly from said proximal end, said resilient anchor portion being deflected by contact with said lumen so that moving said cannula proximally with respect to said localizer causes said anchor portion to project outwardly from said distal end of said cannula and to spring to a size larger than the distance across said lumen; and
an applied insulating coating on said distal portion for helping to insulate said localizer from effects of electrical current from electrosurgical knives used for removing lesions.

2. The electrically insulated breast lesion localizing device of Claim 1 wherein said anchor portion comprises said distal portion being bent in a proximal direction and being capable of springing at least partially back to its original shape when said anchor portion is removed from said cannula.

3. The electrically insulated breast lesion localizing device of Claim 1 wherein said distal portion is bent more than 180° to form a bent loop wherein the longitudinal axes of the portions of the distal portion adjacent to said bent portion cross at an angle of between 5° and 85° measured when said localizer is outside said cannula.

4. The electrically insulated breast lesion localizing device of Claim 1 further includes a hub at said proximal end of said cannula to facilitate handling and positioning said cannula, said hub having a passageway therethrough, said passageway being in communication with said lumen so that said localizer can pass through said passageway and said lumen.

5. The electrically insulated breast lesion localizing device of Claim 1 wherein said insulating coating comprises a plastic material.

6. The electrically insulated breast lesion localizing device of Claim 1 wherein said insulating coating is selected from the group consisting of polyvinyl chloride and polytetrafluoroethylene.

7. The electrically insulated breast lesion localizing device of Claim 1 wherein said localizer is made of metal wire.

8. The electrically insulated breast lesion localizing device of Claim 7 wherein said wire has a diameter within the range of about 0.2mm to 0.3mm.

9. The electrically insulated breast lesion localizing device of Claim 7 wherein said wire is stainless steel.

10. The electrically insulated breast lesion localizing device of Claim 1 wherein said cannula has a circularly-shaped cross section.

FIG-1

*FIG-2*

*FIG-3*

FIG-4

## FIG-5

*28*  *6* *6*  *26*  *31*  *A*  *29*  *32*

## FIG-6

*33*  *27*

FIG-7

FIG-8

FIG-9

21

B

L

22

FIG-10

21

B

26

29

L

31

FIG-11

28

H

26 B

L

FIG-12

45

B

51

L

EP 0 395 997 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 774 948 (MARKHAM) * Column 4, lines 63-68; column 5, lines 34-50; figures 3,4 * | 1-10 | A 61 B 17/34<br>A 61 B 19/00 |
| Y | GB-A-2 136 296 (LEWIS) * Page 1, lines 34-39 * | 1-10 | |

TECHNICAL FIELDS SEARCHED (Int. Cl.5)

A 61 B

The present search report has been drawn up for all claims

| Place of search | Date of completion of the search | Examiner |
|---|---|---|
| THE HAGUE | 09-08-1990 | MOERS R.J. |

EPO FORM 1503 03.82 (P0401)